# EUROPÄISCHE PATENTSCHRIFT

(11) **EP 1 630 173 B1**
(45) Veröffentlichungstag und Bekanntmachung des Hinweises auf die Patenterteilung: **04.11.2009**
(21) Anmeldenummer: 05107881.4
(22) Anmeldetag: 29.08.2005
(51) Int. Cl.: C07K 14/535, C07K 1/113

(54) **Verfahren zur Gewinnung von biologisch aktivem humanen G-CSF aus Inclusion Bodies**
Process for recovering biologically active human G-CSF from inclusion bodies
Procédé de récupération du G-CSF humain sous une forme biologiquement active à partir de corps d'inclusion

(30) Priorität: 27.08.2004 DE 102004041639
(43) Veröffentlichungstag der Anmeldung: 01.03.2006
(73) Patentinhaber: Bioceuticals Arzneimittel AG, 61118 Bad Vilbel (DE)
(72) Erfinder: Blaschke, Ulrich Kurt, 65185, Wiesbaden (DE); Dietrich, Arndt, 08468, Reichenbach (DE); Janowski, Bernhard, 06118, Halle (DE); Schäffner, Jörg, 06198, Salzmünde (DE)
(74) Vertreter: Neuefeind, Regina

(56) Entgegenhaltungen:
- WO-A-01/87925
- WO-A-02/12292
- WO-A-88/08003
- WO-A-93/03134
- WO-A-02/057296
- WO-A-2005/033307
- FISCHER B ET AL: "ISOLATION, RENATURATION, AND FORMATION OF DISULFIDE BONDS OF EUKARYOTIC PROTEINS EXPRESSED IN ESCHERICHIA COLI AS INCLUSION BODIES" BIOTECHNOLOGY AND BIOENGINEERING, WILEY & SONS, HOBOKEN, NJ, US, Bd. 41, Nr. 1, 5. Januar 1993 (1993-01-05), Seiten 3-13, XP000605146 ISSN: 0006-3592
- HALENBECK R ET AL: "RENATURATION AND PURIFICATION OF BIOLOGICALLY ACTIVE RECOMBINANT HUMAN MACROPHAGE COLONY-STIMULATING FACTOR EXPRESSED IN E. COLI" BIO/TECHNOLOGY, NATURE PUBLISHING CO. NEW YORK, US, Bd. 7, Nr. 7, 1. Juli 1989 (1989-07-01), Seiten 710-715, XP000034182 ISSN: 0733-222X
- KAZUYA YAMANISHI ET AL: "RENATURATION, PURIFICATION, AND CHARACTERIZATION OF HUMAN TRUNCATED MACROPHAGE COLONY-STIMULATING FACTOR EXPRESSED IN ESCHERICHIA COLI" JOURNAL OF BIOCHEMISTRY, JAPANESE BIOCHEMICAL SOCIETY / OUP, TOKYO, JP, Bd. 109, Nr. 3, 1. März 1991 (1991-03-01), Seiten 404-409, XP000145569 ISSN: 0021-924X
- BELEW M ET AL: "Purification of recombinant human granulocyte-macrophage colony-stimulating factor from the inclusion bodies produced by transformed Escherichia coli cells." JOURNAL OF CHROMATOGRAPHY. A. 9 SEP 1994, Bd. 679, Nr. 1, 9. September 1994 (1994-09-09), Seiten 67-83, XP002357947 ISSN: 0021-9673

## Beschreibung

Die vorliegende Erfindung betrifft ein Verfahren zur Gewinnung von biologisch aktivem humanen G-CSF aus Einschlusskörpem, sog. Inclusion Bodies, das dadurch gekennzeichnet ist, dass beim Solubilisieren des G-CSF als Reduktionsmittel reduziertes Glutathion und zum Rückfalten des G-CSF als Redoxsystem ein Gemisch aus reduziertem und oxidiertem Glutathion verwendet wird.

G-CSF (Granulozyten Kolonie-stimulierender Faktor) gehört zur Gruppe der Koloniestimulierenden Faktoren, die die Differenzierung und Proliferation von hämatopoetischen Vorläuferzellen und die Aktivierung von Neutrophilen regulieren. Aufgrund dieser Eigenschaften hat G-CSF Anwendung in verschiedenen medizinischen Bereichen gefunden, wie z.B. der Rekonstitution normaler Blutzellpopulationen nach der Chemotherapie oder Bestrahlung, oder zur Stimulation der Immunantwort gegenüber infektiösen Pathogenen. Außerdem wird es zur Behandlung bestimmter Leukämien (Bronchud et al. (1987) Br. J. Cancer 56: 809-813; Morstyn et al. (1988) Lancet 1: 667-672) und bei Knochenmarktransplantationen verwendet.

Wegen der vielfältigen Einsatzmöglichkeiten für G-CSF muss dieses in ausreichend hohen Mengen zur Verfügung gestellt werden. Durch die Erstbeschreibung der DNA-Sequenz von G-CSF im Jahre 1986 wurde die gentechnologische Herstellung ermöglicht. Das erste G-CSF-Präparat, das aus rekombinantem G-CSF hergestellt wurde, wurde 1991 in Deutschland zugelassen und wird unter dem Handelsnamen Neupogen® von der Firma Amgen hergestellt und vertrieben.

Das G-CSF-Protein enthält eine einzige O-Glykosylierungsstelle, wobei die Glykosylierung des Proteins die Stabilität und Aktivität des Proteins nicht beeinflusst. Daher ist es möglich, das rekombinante G-CSF unter Verwendung geeigneter Expressionsvektoren sowohl in Bakterien- als auch in Säugerzellen herzustellen. Die nicht-glykosylierte Form von G-CSF aus Bakterienzellen wird als Filgrastim bezeichnet und seine Produktion ist beschrieben u.a. in EP-A-0 237 545, wogegen die glykosylierte Form von G-CSF aus Säugerzellen als Lenograstim bezeichnet wird, dessen Produktion bspw. in EP-A-0 169 566 beschrieben wurde.

Die Produktion in prokaryotischen Zellen ist gegenüber der Produktion in Säugerzellen bevorzugt, da einfachere Expressionssysteme und Kulturbedingungen verwendet werden können. Ein häufig auftretendes Problem bei der Herstellung von rekombinanten Proteinen in prokaryotischen Zellen ist jedoch die Bildung von schwerlöslichen intrazellulären Aggregaten von denaturierten Formen des exprimierten Proteins, den so genannten Inclusion Bodies, die teilweise eine Sekundärstruktur aufweisen und im Zytoplasma der Bakterienzellen aufgefunden werden können.

Die Bildung dieser Inclusion Bodies führt dazu, dass die Proteine nach der Isolierung der Inclusion Bodies durch Zentrifugation bei mäßiger Geschwindigkeit mit Hilfe geeigneter Mittel solubilisiert und renaturiert werden müssen, um ihre aktive Konfiguration zu erhalten. Dabei ist die Konkurrenzreaktion zwischen einer Überführung des denaturierten Proteins in das richtige Faltungsintermediat und einer Aggregation von mehreren Proteinmolekülen ein wesentlicher, die Ausbeute an renaturiertem Protein limitierender Faktor.

Im Stand der Technik sind bereits verschiedene Verfahren beschrieben worden, mit denen ein in Inclusion Bodies enthaltenes Protein in seine aktive Form überführt werden kann.

EP-A-0 512 087 beschreibt ein Verfahren zur Solubilisierung und Renaturierung von denaturiertem Protein, wobei das Protein mit einem Tris-Puffer behandelt wird, der eine Konzentration von mindestens 400 mmol/l an Tris-Base oder einem Salz von Tris aufweist.

In EP-A-0 719 860 wird die Isolierung und Aufreinigung von G-CSF beschrieben, wobei das G-CSF in Anwesenheit eines Denaturierungsmittels und eines Oxidationsmittels wie Kupfersulfat, das die Luftoxidation katalysiert, solubilisiert und oxidiert wird, bevor das Denaturierungsmittel entfernt wird und das G-CSF einer Ionenaustauschchromatographie unterworfen wird. Dieses Verfahren führt dazu, dass etwa 70% der G-CSF-Moleküle zur korrekten Monomerform oxidiert werden, ein Drittel der Thiolgruppen wird allerdings nicht in die gewünschte Form überführt. Zusätzlich hängt die Effizienz der Luftoxidation von der Oberfläche ab und ist daher schwierig zu kontrollieren.

EP-B-0 364 926 beschreibt ein Verfahren zur Aktivierung von gentechnologisch hergestellten, in Prokaryonten exprimierten biologisch aktiven Proteinen durch Solubilisierung und nachfolgende Reaktivierung, wobei eine Proteinkonzentration von 1 bis 1000 mg/ml eingesetzt wird und zwischen der Solubilisierung und der Reaktivierung eine Dialyse durchgeführt wird. Neben der aufwändigen Durchführung ist ein Hauptnachteil dieses Verfahrens, dass durch die Dialyse zwischen der Solubilisierung und der Reaktivierung Ausbeuteverluste nicht vermieden werden können.

In EP-A-0 219 874 wird ein Verfahren zur Aktivierung von durch Expression in Prokaryonten gentechnologisch hergestellten eukaryontischen Proteinen beschrieben, bei dem die Proteine unter denaturierenden und reduzierenden Bedingungen solubilisiert werden, bevor das Reduktions-/Denaturierungsmittel abgetrennt und die Proteine unter oxidierenden Bedingungen reaktiviert werden. Auch bei diesem Verfahren findet also zwischen der Solubilisierung und der Reaktivierung ein Abtrennungsschritt statt, der mit Ausbeuteverlusten einhergeht.

Schließlich beschreibt WO O1/87925 ein Verfahren zur Solubilisierung und Rückfaltung eines unlöslichen Proteins, wobei das unlösliche Protein solubilisiert wird, indem es mit einem Denaturierungsmittel, einem Reduktionsmittel und einem Cysteinblockierungsmittel behandelt und anschließend durch die Reduktion der Konzentrationen des Denaturierungsmittels und des Reduktionsmittels rückgefaltet wird. Ein Beispiel, das sich mit der Rückfaltung und Solubilisierung von G-CSF beschäftigt, verwendet zur Solubilisierung Harnstoff und Cystein bzw. DTT und zur Rückfaltung Kupfersulfat.

Aufgabe der vorliegenden Erfindung ist es, ein Verfahren zur Gewinnung von biologisch aktivem humanen G-CSF aufzuzeigen, mit dem G-CSF in hoher Reinheit und hoher Ausbeute durch ein möglichst einfaches Verfahren gewonnen werden kann, wobei zwischen der Solubilisierung und der Rückfaltung keine zusätzlichen Verfahrensschritte, insbesondere keine Abtrennung des Reduktionsmittels, stattfinden sollen, sondern die Rückfaltung durch Verdünnung des Solubilisierungspuffers bewirkt werden soll. Diese und weitere Aufgaben werden durch das in Anspruch 1 angegebene Verfahren gelöst. Bevorzugte Ausführungsformen sind in den abhängigen Patentansprüchen beschrieben.

Die Erfindung betrifft somit ein Verfahren zur Gewinnung von biologisch aktivem humanen G-CSF aus Inclusion Bodies, umfassend die Schritte:
a) Solubilisieren des in den Inclusion Bodies enthaltenen G-CSF mit einem Solubilisierungspuffer, der ein Denaturierungsmittel und reduziertes Glutathion in äquimolaren Mengen enthält;
b) Rückfalten des G-CSF durch Verdünnen des Solubilisierungsansatzes mit einem Rückfaltungspuffer, der reduziertes und oxidiertes Glutathion enthält; und
c) Reinigen des rückgefalteten G-CSF durch mindestens einen Chromatographieschritt.

Ein Vorteil dieses Verfahrens ist, dass das Reduktionsmittel nach der Solubilisierung nicht abgetrennt wird, sondern in der Proteinlösung verbleibt. Dadurch, dass ein aufwändiger Abtrennungsschritt vermieden wird, können Ausbeuteverluste des rekombinanten Proteins begrenzt werden. Dies wird vor allem dadurch ermöglicht, dass sowohl im Solubilisierungspuffer als auch im Rückfaltungspuffer reduziertes Glutathion enthalten ist.

In einer bevorzugten Ausführungsform handelt es sich bei dem mindestens einen Chromatographieschritt um eine Kationenaustauschchromatographie.

Mit dem erfindungsgemäßen Verfahren wird eine Ausbeute von rekombinantem G-CSF von 25-35%, bezogen auf das mit der Bradford-Methode bestimmte Gesamtprotein im Solubilisat erhalten. Bezogen auf die Met-G-CSF-Menge im Solubilisat wird eine Faltungsausbeute von etwa 50-70% erreicht, wie durch rpHPLC bestimmt werden kann.

Das durch das erfindungsgemäße Verfahren gewonnene G-CSF weist nach dem ersten Chromatographieschritt eine Reinheit von mehr als 80% auf, die durch nachfolgende Chromatographieschritte gesteigert werden kann.

Unter "biologisch aktivem humanen G-CSF" wird erfindungsgemäß verstanden, dass das durch das erfindungsgemäße Verfahren hergestellte G-CSF in der Lage ist, die Differenzierung und Proliferation von hämatopoetischen Vorläuferzellen zu fördern und die Aktivierung von reifen Zellen des hämatopoetischen Systems zu bewirken. Daher eignet sich das G-CSF, das durch das erfindungsgemäße Verfahren gewonnen wurde, zur Behandlung von Indikationen, bei denen die Verabreichung von G-CSF vorteilhaft ist. Es versteht sich, dass der Begriff "biologisch aktives humanes G-CSF" auch Mutanten und Modifikationen von G-CSF einschließt, deren Aminosäuresequenz gegenüber der Wildtyp-Sequenz verändert ist, die aber eine ähnliche biologische Aktivität wie das Wildtyp-G-CSF aufweisen, wie z.B. die in WO 01/87925 und EP 0 456 200 beschriebenen. Gleiches gilt für G-CSF-Konjugate.

Der Begriff "Inclusion Bodies" bezeichnet intrazelluläre unlösliche, kompakte Aggregate aus inkorrekt gefaltetem oder teilweise korrekt gefaltetem rekombinant exprimierten Protein, die durch Zentrifugation isoliert werden können.

Unter "Solubilisieren" wird erfindungsgemäß verstanden, dass das G-CSF, das die Inclusion Bodies bildet, gelöst wird, indem es mit einem Denaturierungsmittel und reduziertem Glutathion behandelt wird, wodurch inter- und intramolekulare Wechselwirkungen, die im nativen Protein nicht auftreten, gebrochen werden. Dadurch entsteht eine monomolekulare Dispersion des rekombinanten Proteins.

Denaturierungsmittel sind Mittel, in deren Gegenwart die native Konformation von Proteinen nicht erhalten bleibt. Daher ist die biologische Aktivität der Proteine in der Gegenwart von Denaturierungsmitteln verringert bzw. nicht vorhanden. Zur Verwendung in Solubilisierungspuffern geeignete Denaturierungsmittel schließen ein Harnstoff, Guanidin-HCl, Arginin, Natriumthiocyanat, pH-Extreme (verdünnte Säuren oder Basen), Detergenzien (z.B. SDS, Sarkosyl), Salze (Chloride, Nitrate, Thiocyanate, Trichloroacetate), chemische Derivatisierung (Sulfitolyse, Reaktion mit Citraconanhydrid), Lösungsmittel (2-Amino-2-methyl-1-propanol oder andere Alkohole, DMSO, DMS) oder starke Anionenaustauschharze wie etwa Q-Sepharose.

Geeignete Konzentrationen von Harnstoff sind 1-9 mol/l, bevorzugt 5-9 mol/l. Geeignete Konzentrationen von Guanidin-HCl sind 1-8 mol/l, bevorzugt 4-8 mol/l. Bevorzugt wird im Verfahren der vorliegenden Erfindung Guanidin-HCl als Denaturierungsmittel verwendet. Besonders bevorzugt beträgt die Konzentration des Guanidin-HCl 5,0 bis 7,0 mol/l, am meisten bevorzugt 6,0 mol/l.

Des Weiteren enthält der erfindungsgemäße Solubilisierungspuffer neben dem Denaturierungsmittel reduziertes Glutathion in einer Konzentration von 10-200 mmol/1, bevorzugt von 40-150 mmol/1, besonders bevorzugt von 60-120 mmol/1 und am meisten bevorzugt von 100 mmol/1.

Weiterhin ist es bevorzugt, dem Solubilisierungspuffer Chelatbildner, wie etwa EDTA oder DMAE, zuzusetzen, die Metallionen komplexieren können. Lägen diese Metallionen in freier Form vor, bestünde die Möglichkeit, dass diese die Luftoxidation des eingesetzten Reduktionsmittels katalysieren könnten, das dann nicht mehr zur Reduktion des Proteins zur Verfügung stehen würde.

Als eigentliche Pufferkomponente des Solubilisierungspuffers eignen sich insbesondere Tris oder Phosphat, die einen leicht basischen pH-Wert aufweisen sollten, um die Reduktion von Disulfidbrücken zu ermöglichen.

Um eine möglichst effiziente und vollständige Solubilisierung zu gewährleisten, muss ein geeignetes Verhältnis von Inclusion Bodies und Solubilisierungspuffer verwendet werden. Bevorzugt werden pro Gramm Inclusion Bodies 4-20 ml Solubilisierungspuffer, besonders bevorzugt 7-15 ml Solubilisierungspuffer und am meisten bevorzugt 9 ml Solubilisierungspuffer eingesetzt.

Durch die oben beschriebene Solubilisierung werden Proteinkonzentrationen in den Solubilisaten von jeweils 20-40 mg/ml, bevorzugt von 25-35 mg/ml und am meisten bevorzugt von etwa 30 mg/ml, bezogen auf den Met-G-CSF-Anteil, erreicht.

Das Solubilisat muss nach der Solubilisierung und vor der Rückfaltung nicht angesäuert werden, da auf eine Entfernung des Reduktionsmittels verzichtet wird.

Nach der Solubilisierung der Inclusion Bodies muss das Protein in seine aktive Form zurückgefaltet werden. Dazu muss das Protein seine native Konformation annehmen und seine nativen Disulfidbrücken ausbilden. Im erfindungsgemäßen Verfahren erfolgt diese Rückfaltung, ohne dass vorher der Solubilisierungspuffer abgetrennt wird, vielmehr wird der Solubilisierungsansatz durch Mischen mit dem Rückfaltungspuffer verdünnt. Dadurch wird die Konzentration des Denaturierungsmittels reduziert, so dass das Protein in seine lösliche, biologisch aktive Form renaturieren kann. Durch die Verdünnung des Solubilisierungsansatzes wird ein Ausbeuteverlust verhindert, der auftreten würde, wenn das Denaturierungsmittel durch z.B. Dialyse oder Gelfiltration entfernt werden würde.

Der Rückfaltungspuffer enthält reduziertes und oxidiertes Glutathion, vorzugsweise in äquimolaren Mengen. Die Verwendung dieses Redoxsystems aus Disulfid und Thiol ermöglicht die korrekte Bildung der Disulfidbrücken im Protein, indem es die rasche Umbildung von falsch gebildeten Disulfidbrücken fördert. Die Verwendung des Redoxpaares aus reduziertem und oxidiertem Glutathion bietet außerdem den Vorteil, dass die Faltung besser kontrollierbar ist als etwa eine durch Luftoxidation bewirkte Faltung, da die Faltung nicht vom Oberflächen-Volumen-Verhältnis abhängig ist.

Bevorzugt beträgt die Konzentration von reduziertem und oxidiertem Glutathion jeweils 0,2-10 mmol/1, bevorzugt 0,5-5 mmol/1, besonders bevorzugt 1-3 mmol/1 und am meisten bevorzugt 2 mmol/1.

Neben dem Redoxpaar reduziertes und oxidiertes Glutathion enthält der Rückfaltungspuffer vorzugsweise einen Zusatz ausgewählt aus der Gruppe bestehend aus den Denaturierungsmitteln Arginin, Harnstoff und Guanidin-HCl, wobei diese Denaturierungsmittel in einer die Rückfaltung fördernden Konzentration vorliegen. Der Begriff "in einer die Rückfaltung fördernden Konzentration" bedeutet im Sinne der Erfindung, dass die Konzentration der Denaturierungsmittel im Rückfaltungspuffer geringer ist als die Konzentration im Solubilisierungspuffer. Dadurch werden nichtproduktive Faltungsintermediate destabilisiert, was zu ihrer erneuten Entfaltung führt. Die so entfalteten Proteine werden einem weiteren Rückfaltungsschritt zugeführt, in dem sie ihre aktive Konfiguration annehmen können, was eine Erhöhung der Ausbeute an aktivem Protein bewirkt. Außerdem ermöglichen die Denaturierungsmittel die Faltung des Proteins bei höheren Proteinkonzentrationen im Rückfaltungsansatz.

Besonders bevorzugt handelt es sich bei dem Zusatz um Harnstoff. Die Konzentration des Harnstoffs im Rückfaltungspuffer beträgt von 1,5-4,5 mol/l, bevorzugt von 2,0-4,0 mol/l und besonders bevorzugt 3,0 mol/l.

Zusätzlich kann der Rückfaltungspuffer EDTA enthalten, bevorzugt liegt die Konzentration von EDTA im Rückfaltungspuffer bei 1 mM.

In einer Ausführungsform enthält der Rückfaltungspuffer, abgesehen von ggf. Arginin, keine mercaptofreien Aminosäuren, insbesondere enthält er, abgesehen von ggf. Arginin, überhaupt keine Aminosäuren.

Der pH-Wert des Rückfaltungspuffers hat einen umfassenden Einfluss auf die gesamte Faltungsreaktion, da er u.a. Ladungsverhältnisse im Protein bedingt und so die Bildung der nativen Struktur begünstigen, aber auch zur verstärkten Bildung von falsch gefalteten Spezies führen kann. Außerdem beeinflusst der pH-Wert die Geschwindigkeit des Disulfidbrückenaustausches, welcher ein wichtiger Schritt bei der Faltung von Disulfid-verbrückten Proteinen ist. Da die Bildung der Disulfidbrücken in den Proteinen nur im neutralen und im basischen Bereich erfolgen kann, sollte der pH-Wert des Rückfaltungspuffers im Bereich von 7,0 bis 11,0 liegen. Bevorzugt liegt er zwischen 7,5 und 8,0 und am meisten bevorzugt bei 7,5.

Die Effizienz der Rückfaltung wird auch beeinflusst durch die Proteinkonzentration im Faltungsansatz. Für monomere Proteine sind die Faltungsausbeuten in der Regel am günstigsten, wenn die Proteinkonzentration so gering wie möglich ist. Höhere Proteinkonzentrationen begünstigen Nebenreaktionen im Faltungsprozess wie die Aggregation von stärker hydrophoben Intermediaten. Allerdings kann die Proteinkonzentration nicht beliebig gering gewählt werden, da das Volumen der Faltungsreaktion im Produktionsmaßstab aus technischen und Kostengründen begrenzt ist. Im erfindungsgemäßen Verfahren wird daher eine Proteinkonzentration von weniger als 2000 µg/ml, wie bestimmt durch einen Bradford-Assay mit dem Solubilisat, eingesetzt, bevorzugt weniger als 1200 µg/ml und besonders bevorzugt weniger als 700 µg/ml.

Ein weiterer Parameter, der das erfindungsgemäße Verfahren beeinflusst, ist die Temperatur, bei der die Rückfaltung stattfindet. Häufig werden bei niedrigen Temperaturen (weniger als 10°C) bessere Ausbeuten erreicht als bei 20°C oder mehr. Allerdings verlangsamt sich der Faltungsprozess bei geringeren Temperaturen, so dass auch die Faltungszeit auf die verwendete Temperatur abgestimmt werden muss. Im erfindungsgemäßen Verfahren findet die Rückfaltung bei 4°C statt. Die Rückfaltung erfolgt für mindestens 3 Stunden, bevorzugt für mindestens 10 Stunden, besonders bevorzugt für mindestens 12 Stunden und am meisten bevorzugt für mindestens 15 Stunden.

Nach der Solubilisierung und Rückfaltung des G-CSF wird dieses durch mindestens einen Chromatographieschritt aufgereinigt. Vor diesem Schritt kann ggf. eine andere Art der Aufbereitung erfolgen, z.B. eine Filtration, Konzentrierung, Fällung, Ansäuerung und/oder Dialyse. Für G-CSF eignen sich prinzipiell die beiden Chromatographiemethoden Ionenaustausch-Chromatographie und hydrophobe Interaktions-Chromatographie besonders gut. Bevorzugt erfolgt eine Ionenaustausch-Chromatographie, besonders bevorzugt eine Kationenaustauschchromatographie, bei der das G-CSF bei einem bestimmten pH des Puffers durch seine positive Gesamtladung an die Matrix der Kationenaustauschmatrix bindet, während die meisten kontaminierenden Stoffe, wie Nukleinsäuren, Lipopolysaccharide und Proteine, die aus den Wirtszellen stammen, sowie ionische Isomere von G-CSF und veränderte Formen von G-CSF mit anderen pI-Werten nicht binden und durch Waschen entfernt werden können.

Geeignete Kationaustauschmatrices schließen ein, sind aber nicht beschränkt auf, Carboxymethyl (CM)-Cellulose, AG 50 W, Bio-Rex 70, Carboxymethyl (CM)-Sephadex, Sulfopropyl (SP)-Sephadex, Carboxymethyl (CM)-Sepharose CL-6B und Sulfonat (S)-Sepharose. Bevorzugt wird SP-Sepharose XL, erhältlich von Amersham Biosciences, Freiburg, Deutschland, als Matrix für die Kationenaustauschchromatographie verwendet.

Geeignete Matrices und Protokolle zur Durchführung der Kationenaustauschchromatographie kann der Fachmann den Produktinformationen von Anbietern wie Amersham Biosciences (http://www.amershambiosciences.com) oder Bio-Rad (http://www.bio-rad.com) entnehmen.

Geeignete Puffer für die Kationenaustauschchromatographie schließen ein Maleat-, Malonat-, Citrat-, Lactat-, Acetat-, Phosphat-, HEPES- und Bicin-Puffer. Die Konzentration des Puffers liegt bevorzugt zwischen 20 mM und 50 mM. Der pH des Puffers sollte für die Aufreinigung des G-CSF möglichst nicht höher als 8,0, bevorzugt nicht höher als 7,0 sein.

Besonders bevorzugt wird für die Kationenaustauschchromatographie 20 mM Natriumacetat pH 5,0 verwendet, das zum Äquilibrieren und Waschen eingesetzt wird.

Das G-CSF kann nach dem Waschen durch eine Veränderung, im Falle der Kationenaustauschchromatographie durch eine Erhöhung, des pH-Wertes oder eine Erhöhung der Ionenstärke von der Säule eluiert werden.

Bevorzugt wird die Elution durch Erhöhung der Ionenstärke bewirkt. Wenn 20 mM Natriumacetat pH 5,0 zum Äquilibrieren und Waschen verwendet wird, wird zur Elution ein Gemisch von 20 mM Natriumacetat pH 5,0 und 200 mM NaCl eingesetzt.

Weitere geeignete Bedingungen für die Kationenaustauschchromatographie können der einschlägigen Literatur, wie etwa dem Handbuch "Ion Exchange Chromatography-Principles and Methods" von Amersham Biosciences, Freiburg, Deutschland, 2002, entnommen werden.

Auch die hydrophobe Interaktions-Chromatographie kann mit üblichen Matrices durchgeführt werden. Geeignet sind Matrices wie Butyl-, Phenyl- oder Octyl-Sepharose (Amersham Biosciences), Makro-Prep-Methyl oder t-Butyl (Bio-Rad) und Fractogel EMD mit Propyl- oder Phenyl-Liganden (Merck).

Um das G-CSF nach der Rückfaltung chromatographisch reinigen zu können, muss der Faltungsansatz vorher geklärt werden, d.h. es müssen höher molekulare Partikel entfernt werden, bei denen es sich vor allem um Proteinaggregate handelt, die bei der Faltung gebildet wurden. Diese Klärung erfolgt durch Tiefenfiltration, bei der eine lockere Schüttung eines körnigen Materials als Filtermittel dient. Die Feststoffteilchen sind kleiner als die Poren des Filtermittels oder werden durch Adsorption an der inneren Oberfläche der Schüttung zurückgehalten.

Bevorzugt werden für die Tiefenfiltration Celluloseester-Fasern als Filtermedium eingesetzt. Geeignete Filtermaterialien sowie entsprechende Anwendungsvorschriften sind z.B. erhältlich von der Firma Millipore unter den Handelsnamen Millistak Plus C0HC und Millistak + B1HC.

Bevorzugt wird vor der Tiefenfiltration der Faltungsansatz angesäuert, so dass das Filtrat unmittelbar für die Kationenaustauschchromatographie eingesetzt werden kann. Bevorzugt wird der pH-Wert des Faltungsansatzes hierbei auf unter 4,0, besonders bevorzugt auf etwa 3,2 eingestellt.

Zur Bestimmung der Faltungsausbeute kann nach der Gewinnung des G-CSF eine rpHPLC durchgeführt werden, bei der das Protein denaturiert wird. Aufgrund der Disulfidbrücken, die erhalten bleiben, besitzen unterschiedlich Disulfid-überbrückte Spezies häufig unterschiedliche hydrophobe Oberflächen und können damit in der rpHPLC getrennt werden. Deshalb wird mit dieser Methode nur der Nachweis der richtigen Disulfidverbrückung erbracht. Allerdings ist diese ein entscheidendes und für viele kleine und entsprechend verbrückte Proteine bereits ein ausreichendes Kriterium für die richtige Faltung. Als weitere Analysemethode kann auch eine Size Exclusion (SE)-HPLC durchgeführt werden.

Geeignete Materialien und Protokolle zur Durchführung der rpHPLC oder der SE-HPLC kann der Fachmann den Produktinformationen von Anbietern wie Vydac (http://www.vydac.com) oder TOSOH Bioscience (http://www.tosohbiosep.de) entnehmen. Die Bestimmung der Ausbeute an Met-G-CSF ist auch beschrieben in Herman et al. (1996) Pharm. Biotechnol. 9: 303-328. Der genaue Anteil an Met-G-CSF wird dabei durch Integration der Peakfläche und Umrechnung anhand des Extinktionskoeffizienten ermittelt.

Nach der Aufreinigung kann das G-CSF hinsichtlich seiner Menge und seiner Aktivität analysiert werden. Eine qualitative Analyse kann über eine SDS-PAGE-Analyse mit anschließender Coomassie-Brilliant-Blue-Färbung oder eine rpHPLC erfolgen. Als Standard für die Analysen kann ein kommerziell erhältliches G-CSF-Präparat verwendet werden. Zusätzlich kann eine Peptide Map bzw. eine Massenspektroskopie durchgeführt werden. Die Aktivität des aufgereinigten G-CSF kann mit verschiedenen biologischen Testverfahren bestimmt werden, wie sie beispielsweise in Shirafuji et al. (1989) Exp. Hematol. 17(2): 116-119; Oh-Eda et al. (1990) J. Biol. Chem. 265(20): 11432-11435; Stute et al. (1992) Blood 79(11): 2849-2854 und Oshima et al. (2000) Biochem. Biophys. Res. Commun. 267(3): 924-927 beschrieben sind.

Die Erfindung betrifft auch pharmazeutische Zubereitungen, die das erfindungsgemäß gewonnene G-CSF enthalten. Das gewonnene G-CSF kann entweder als Lyophilisat oder in flüssiger Form aufbewahrt werden. Es wird entweder subkutan oder intravenös verabreicht. Geeignete Hilfsstoffe in den Formulierungen des rekombinant exprimierten G-CSF sind etwa Stabilisatoren wie Zucker und Zuckeralkohole, Aminosäuren sowie Tenside wie z.B. Polysorbat 20/80 sowie geeignete Puffersubstanzen. Beispiele für Formulierungen sind beschrieben in EP 0 674 525, EP 0 373 679 sowie EP 0 306 824, siehe auch Handelsprodukte Neupogen® und Granocyte in ROTE LISTE 2004.

Die folgenden Beispiele sollen die Erfindung erläutern, ohne sie einzuschränken.

### Beispiele:

Das humane G-CSF wurde unter der Kontrolle eines IPTG-induzierbaren Promotors in E. *coli*-Zellen exprimiert. Beispiele für geeignete Expressionssysteme können z.B. dem Laborhandbuch Sambrook und Russell, Molecular Cloning― A Laboratory Manual, 3. Auflage 2001, Cold Spring Harbor Laboratory Press, Cold Spring Harbor, NY, Kapitel 15, oder gängigen Herstellerprotokollen, z.B. von Promega oder Stratagene, entnommen werden.

Die Fermentation erfolgte nach Standardprotokollen, wie sie in der Patent- und wissenschaftlichen Literatur beschrieben sind, in einem zweistufigen Prozess umfassend eine Batch-Kultivierung und eine Fed-Batch-Kultivierung. Die Bakterien wurden für 17 bis 18 Stunden kultiviert, bevor sie durch Zugabe von 1 mM IPTG zur Bildung des rekombinanten G-CSF angeregt wurden. Die Induktionsdauer betrug 4,0 Stunden.

Die Ernte der Bakterien erfolgte durch Becherzentrifugation für 20 Minuten bei 5000 g und 4°C. Nach der Zentrifugation wurde der Überstand verworfen und die Zellen mit Puffer (20 mM Natriumphosphat, pH 7,0; 1 mM EDTA) wieder auf das Fermentationsvolumen aufgefüllt, bevor sie durch drei Passagen bei 800 bar aufgeschlossen wurden. Anschließend wurde das Lysat durch Separation (CSA1-Separator, Westfalia, Oelde) geklärt.

### 1. Konzentrieren der Inclusion Bodies

Prinzipiell kann die durch die Ernte gewonnene Suspension der Inclusion Bodies direkt für die nachfolgende Solubilisierung eingesetzt werden. Allerdings ist in diesem Fall die maximal erreichbare Proteinkonzentration im Solubilisat stark limitiert, was zur Einschränkung während der Faltung führen kann. Daher sollte die Suspension der Inclusion Bodies nach der Ernte und dem Waschen durch Zentrifugation konzentriert werden, um eine hohe Proteinkonzentration im Solubilisat zu erreichen.

Die Suspension der Inclusion Bodies wurde für 20 Minuten bei 10.000g in einer Becherzentrifuge zentrifugiert. Die durch die Zentrifugation gewonnene Paste der Inclusion Bodies kann für mindestens 12 Wochen bei ―20°C gelagert werden.

### 2. Solubilisierung

Um größere Pellets von Inclusion Bodies in einer relativ kurzen Zeit effektiv solubilisieren zu können, ist außerdem eine mechanische Zerkleinerung dieser Pellets, etwa durch Ultraturrax-Behandlung, notwendig. Die durch Zentrifugation erhaltene Paste der Inclusion Bodies wurde gewogen, mit 9,0 ml Solubilisierungspuffer (30 mM Tris, 1 mM EDTA, 6,0 M Guanidin-HCl, 100 mM reduziertes Glutathion, pH 8,0) pro Gramm Inclusion Bodies versetzt und durch Ultraturrax-Behandlung zerkleinert. Der Ansatz wurde gründlich gevortext und dann auf einem Roller-Mixer bei Raumtemperatur für etwa 2 Stunden inkubiert.

### 3. Rückfaltung

Die Proteinkonzentration im Solubilisat wurde mit der Methode nach Bradford mit BSA als Standardprotein bestimmt. Für die Faltung wurde die Menge Solubilisat zum Rückfaltungspuffer (30 mM Tris, 2 mM reduziertes Glutathion, 2 mM oxidiertes Glutathion, 3,0 M Harnstoff, pH 7,5) zugegeben, die benötigt wurde, um in einer gewünschten Menge Puffer eine Proteinkonzentration von 700 mg/ml zu erzielen. Die entsprechende Menge an Solubilisat wurde unter Rühren mit einem Magnetrührer langsam und gleichmäßig zugegeben, um lokal erhöhte Konzentrationen an Solubilisat bzw. Protein zu vermeiden. Die Zuflussgeschwindigkeit und die Art der Durchmischung kann für das jeweilig verwendete Volumen des Solubilisierungsansatzes angepasst werden. Nach abgeschlossener Zugabe des Solubilisates wurde der Ansatz für mindestens 12 Stunden bei 4°C inkubiert. Während dieser Zeit war ein weiteres Mischen nicht erforderlich.

### 4. Tiefenfiltration

Der Rückfaltungsansatz wurde zunächst mit 2 M Zitronensäure auf einen pH-Wert von 3,2 eingestellt und mit einem Millistak B1HC-Filter (Firma Millipore, Schwalbach) nach Herstellerangaben geklärt.

### 5. Kationenaustauschchromatographie

Das Filtrat wurde anschließend einer Ionenaustauschchromatographie unterzogen. Als Matrix wurde die SP-Sepharose XL von Amersham Biosciences verwendet, die mit 20 mM Natriumacetat, pH 5,0 äquilibriert wurde. Die Proben wurden auf die Säule geladen und mit 1,5 Säulenvolumen Waschpuffer (20 mM Natriumacetat, pH 5,0) gewaschen. Anschließend wurde das G-CSF mit 3 Säulenvolumen Elutionspuffer (20 mM Natriumacetat, pH 5,0; 200 mM NaCl) stufenweise eluiert.

### 6. Bestimmung der Reinheit und der Ausbeute

Die Ausbeute der Aufreinigung insgesamt wurde durch rpHPLC und SDS-PAGE-Analyse bestimmt. Sie lag bei etwa 35%, bezogen auf das Gesamtprotein im Solubilisat, wie es im Bradford-Assay bestimmt wurde. Die Reinheit des Eluats lag bei mehr als 80%.

### 7. Bestimmung der biologischen Aktivität

Die Aktivität des durch das erfindungsgemäße Verfahren gewonnenen G-CSF wurde durch einen Bioassay bestimmt und mit der eines Standards, kommerziell erhältlichem G-CSF (Neupogen®), verglichen. Dazu wurde die Mauszelllinie NFS-60 verwendet, die G-CSFresponsiv ist. Diese Zelllinie wurde in RPMI 1640-Medium (Firma Bachem, Heidelberg) kultiviert, das 1,5 g/1 Natriumbicarbonat, 4,5 g/1 Glucose, 10 mM Hepes und 1,0 mM Natriumpyruvat enthielt und mit 2 mM Glutamin, 10% FCS, 0,05 mM 2-Mercaptoethanol und 60 ng/ml G-CSF supplementiert worden war.

Für den Aktivitätstest wurden die Zellen zweimal mit Medium ohne G-CSF gewaschen, in einer Konzentration von 2 x 10⁴ Zellen pro Napf in 96-Napf-Platten ausgesät und für drei Tage bei 37°C und 4,5% CO₂ mit verschiedenen Konzentrationen des aufgereinigten G-CSF und des Standards inkubiert. Danach wurden sie mit XTT-Reagenz angefärbt und die Absorption bei 450 nm in einem Mikrotiterplattenlesegerät gemessen. Es zeigte sich, dass die Zellen, die mit dem erfindungsgemäß aufgereinigten G-CSF behandelt worden waren, genauso gut wuchsen wie die Zellen, die mit dem Standard behandelt worden waren, weshalb von einer gleichen biologischen Aktivität der beiden G-CSF-Proben ausgegangen werden kann.

## Patentansprüche

1. Verfahren zur Gewinnung von biologisch aktivem humanen G-CSF aus Inclusion Bodies, umfassend die Schritte:
a) Solubilisieren des in den Inclusion Bodies enthaltenen G-CSF mit einem Solubilisierungspuffer, der ein Denaturierungsmittel und reduziertes Glutathion enthält;
b) Rückfalten des G-CSF durch Verdünnen des Solubilisierungsansatzes mit einem Rückfaltungspuffer, der reduziertes und oxidiertes Glutathion in äquimolaren Mengen enthält; und
c) Reinigen des rückgefalteten G-CSF durch mindestens einen Chromatographieschritt.

2. Verfahren nach Anspruch 1, wobei das Denaturierungsmittel Guanidin-HCl ist.

3. Verfahren nach Anspruch 2, wobei die Konzentration des Guanidin-HCl 4,0 bis 8,0 mol/l beträgt.

4. Verfahren nach einem der vorangehenden Ansprüche, wobei die Konzentration des reduzierten Glutathions im Solubilisierungspuffer 10 bis 200 mmol/l beträgt.

5. Verfahren nach einem der vorangehenden Ansprüche, wobei der Solubilisierungspuffer des Weiteren einen Chelatbildner enthält.

6. Verfahren nach einem der vorangehenden Ansprüche, wobei pro Gramm Inclusion Bodies 4 bis 20 ml Solubilisierungspuffer eingesetzt werden.

7. Verfahren nach einem der vorangehenden Ansprüche, wobei zwischen der Solubilisierung und der Rückfaltung keine Abtrennung des Solubilisierungspuffers erfolgt.

8. Verfahren nach einem der vorangehenden Ansprüche, wobei der Rückfaltungspuffer einen Zusatz ausgewählt aus der Gruppe bestehend aus Arginin, Harnstoff und Guanidin-HCl in einer die Rückfaltung fördernden Konzentration enthält.

9. Verfahren nach Anspruch 8, wobei der Zusatz Harnstoff ist.

10. Verfahren nach Anspruch 8 oder 9, wobei der Rückfaltungspuffer Harnstoff in einer Konzentration von 1,5 bis 4,5 mol/l enthält.

11. Verfahren nach einem der vorangehenden Ansprüche, wobei die Konzentration von reduziertem und oxidiertem Glutathion jeweils 0,2 bis 10 mmol/l beträgt.

12. Verfahren nach einem der vorangehenden Ansprüche, wobei die für die Rückfaltung eingesetzte Proteinkonzentration unter 2000 µg/ml liegt.

13. Verfahren nach einem der vorangehenden Ansprüche, wobei das Rückfalten bei 4°C für mindestens drei Stunden erfolgt.

14. Verfahren nach einem der vorangehenden Ansprüche, wobei es sich bei dem mindestens einen Chromatographieschritt um eine Ionenaustauschchromatographie handelt.

15. Verfahren nach Anspruch 14, wobei es sich bei der Ionenaustauschchromatographie um eine Kationenaustauschchromatographie handelt.

16. Verfahren nach Anspruch 15, wobei der Faltungsansatz vor der Kationenaustauschchromatographie angesäuert wird.

17. Verfahren nach Anspruch 15 oder 16, wobei die Elution des G-CSF bei einem pH-Wert von 4,0-6,0 erfolgt.

18. Verfahren nach einem der vorangehenden Ansprüche, wobei nach der Rückfaltung, aber vor dem mindestens einen Chromatographieschritt eine Tiefenfiltration durchgeführt wird.

## Claims

1. A process for obtaining biologically active human G-CSF from inclusion bodies comprising the steps:
(a) solubilising the G-CSF contained in the inclusion bodies with a solubilisation buffer containing a denaturing agent and reduced glutathione;
(b) refolding the G-CSF by diluting the solubilisate with a refolding buffer containing reduced and oxidised glutathione in equimolar amounts; and
(c) purifying the refolded G-CSF by at least one chromatography step.

2. The process according to claim 1, wherein the denaturing agent is guanidine-HCl.

3. The process according to claim 2, wherein the concentration of guanidine-HCl is 4.0 to 8.0 mol/L.

4. The process according to any of the preceding claims, wherein the concentration of the reduced glutathione in the solubilisation buffer is 10 to 200 mmol/L.

5. The process according to any of the preceding claims, wherein the solubilisation buffer further contains a chelating agent.

6. The process according to any of the preceding claims, wherein 4 to 20 mL of solubilisation buffer per gram of inclusion bodies are used.

7. The process according to any of the preceding claims, wherein the solubilisation buffer is not removed between solubilisation and refolding.

8. The process according to any of the preceding claims wherein the refolding buffer contains an additive selected from the group consisting of arginine, urea and guanidine-HCl in a refolding promoting concentration.

9. The process according to claim 8, wherein the additive is urea.

10. The process according to claim 8 or 9, wherein the refolding buffer contains urea in a concentration of 1.5 to 4.5 mol/L.

11. The process according to any of the preceding claims, wherein the concentration of reduced and oxidised glutathione is 0.2 to 10 mmol/L each.

12. The process according to any of the preceding claims, wherein the protein concentration used for refolding is below 2000 µg/mL.

13. The process according to any of the preceding claims, wherein refolding is carried out at 4 °C for at least 3 hours.

14. The process according to any of the preceding claims, wherein the at least one chromatography step is an ion-exchange chromatography.

15. The process according to claim 14, wherein the ion-exchange chromatography is a cation exchange chromatography.

16. The process according to claim 15, wherein the refolding batch is acidified prior to the cation exchange chromatography.

17. The process according to claim 15 or 16, wherein the elution of G-CSF takes place at a pH-value of 4.0 - 6.0.

18. The process according to any of the preceding claims, wherein after refolding but prior to the at least one chromatography step a deep bed filtration is carried out.

## Revendications

1. Procédé pour l'obtention du G-CDF humain biologiquement actif à partir des corps d'inclusion, comprenant les étapes suivantes :
a) solubiliser, avec un tampon solubilisant contenant un agent dénaturant et du glutathion réduit, le G-CSF inclus dans les corps d'inclusion ;
b) replier le G-CSF par dilution la charge à solubiliser avec un tampon de repliage qui contient du glutathion réduit et oxydé en quantités équimolaires ; et
c) purifier le glutathion replié par au moins une étape de chromatographie.

2. Procédé selon la revendication 1, dans lequel l'agent dénaturant est du guanidine-HCl.

3. Procédé selon la revendication 2, dans lequel la concentration du guanidine-HCl est 4,0 à 8,0 mol/l.

4. Procédé selon l'une des revendications précédentes, dans lequel la concentration du guanidine-HCl réduit dans le tampon solubilisant est 10 à 200 mmol/l.

5. Procédé selon l'une des revendications précédentes, dans lequel le tampon solubilisant contient en outre un chélateur.

6. Procédé selon l'une des revendications précédentes, dans lequel on utilise 4 à 20 ml du tampon solubilisant par gramme des corps d'inclusion.

7. Procédé selon l'une des revendications précédentes, dans lequel il n'y a aucune séparation du tampon solubilisant entre la solubilisation et le repliage.

8. Procédé selon l'une des revendications précédentes, dans lequel le tampon de repliage contient un ajout sélectionné parmi le group consistant en l'arginine, l'urée et le guanidine-HCl dans une concentration favorisant le repliage.

9. Procédé selon la revendication 8, dans lequel l'ajout est de l'urée.

10. Procédé selon la revendication 8 ou 9, dans lequel le tampon de repliage contient de l'urée dans une concentration de 1,5 à 4,5 mol/l.

11. Procédé selon l'une des revendications précédentes, dans lequel la concentration en glutathion réduit et oxydé est 0,2 à 10 respectivement.

12. Procédé selon l'une des revendications précédentes dans lequel la concentration de protéine utilisée pour le repliage est au-dessous de 2000 µg/ml.

13. Procédé selon l'une des revendications précédentes, dans lequel le repliage est réalisé pendant au moins trois heures à 4 °C.

14. Procédé selon l'une des revendications précédentes, dans lequel il s'agit chez ladite au moins une étape de chromatographie d'une chromatographie à échange d'ions.

15. Procédé selon la revendication 14, dans lequel il s'agit chez la chromatographie à échange d'ions d'une chromatographie à échange de cations.

16. Procédé selon la revendication 15, dans lequel la charge de pliage est acidifiée avant la chromatographie à échange de cations.

17. Procédé selon la revendication 15 ou 16, dans lequel l'élution du G-CSF se fera chez une valeur de pH de 4,0-6,0.

18. Procédé selon l'une des revendications précédentes, dans lequel, après le repliage mais avant la dite au moins une étape de chromatographie, une filtration en profondeur est réalisée.
